(19) Europäisches Patentamt
European Patent Office
Office européen des brevets

(11) Veröffentlichungsnummer: **0 054 786**
**B1**

(12) # EUROPÄISCHE PATENTSCHRIFT

(45) Veröffentlichungstag der Patentschrift: **20.03.85**

(51) Int. Cl.⁴: **C 07 J 5/00**, A 61 K 31/57 // C07J21/00, C12P33/08

(21) Anmeldenummer: **81110137.7**

(22) Anmeldetag: **04.12.81**

(54) **Neue 6-alpha-Methylhydrocortison-Derivate, ihre Herstellung und Verwendung.**

(30) Priorität: **23.12.80 DE 3049400**

(43) Veröffentlichungstag der Anmeldung: **30.06.82 Patentblatt 82/26**

(45) Bekanntmachung des Hinweises auf die Patenterteilung: **20.03.85 Patentblatt 85/12**

(84) Benannte Vertragsstaaten: **AT BE CH DE FR GB IT LI LU NL SE**

(56) Entgegenhaltungen:
**US - A - 3 141 876**
**US - A - 3 176 032**

**JOURNAL OF THE AMERICAN CHEMICAL SOCIETY, Band 78, 5. Dezember 1956, Seiten 6213-6214, Washington, D.C., U.S.A., G.B. SPERO et al.: "Adrenal hormones and related compounds. IV. 6-Methyl steroids"**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT Berlin und Bergkamen, Müllerstrasse 170/178 Postfach 65 03 11, D-1000 Berlin 65 (DE)**

(72) Erfinder: **Annen, Klaus, Dr., Seegefelder Strasse 194, D-1000 Berlin 20 (DE)**
Erfinder: **Petzoldt, Karl, Dr., Flachsweg 10, D-1000 Berlin 38 (DE)**
Erfinder: **Laurent, Henry, Dr., Glambecker Weg 21, D-1000 Berlin 28 (DE)**
Erfinder: **Wiechert, Rudolf, Prof. Dr., Petzower Strasse 8a, D-1000 Berlin 39 (DE)**
Erfinder: **Hofmeister, Helmut Dr., Weislingenstrasse 4, D-1000 Berlin 28 (DE)**
Erfinder: **Wendt, Hans, Dr., Ernst-Ring-Strasse 14, D-1000 Berlin 38 (DE)**
Erfinder: **Albring, Manfred, Dr., Am Dachsbau 34a, D-1000 Berlin 27 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 054 786 B1

ACTORUM AG

**Beschreibung**

Die Erfindung betrifft den in den Patentansprüchen gekennzeichneten Gegenstand.

Die neuen 6α-Methylhydrocortison-Derivate der allgemeinen Formel I gemäss Patentanspruch 1 können als 2 bis 6 Kohlenstoffatome enthaltende 1-Oxoalkylgruppen $R_1$ und $R_2$ beispielsweise eine Acetylgruppe, eine Propionylgruppe, eine Butyrylgruppe, eine Isobutyrylgruppe, eine Valerylgruppe, eine 3-Methylbutyrylgruppe, eine Trimethylacetylgruppe oder eine Hexanoylgruppe tragen.

6α-Methylhydrocortison und 21-Ester dieser Verbindungen sind seit langem bekannt (J. Amer. Chem. Soc., 78, 1956, 6213 f). Diesen Substanzen kommt seit langem eine erhebliche Bedeutung als Zwischenprodukte zur Synthese von 6α-Methyl-prednisolon zu; als pharmakologisch wirksame Substanzen wurde das 6α-Methylhydrocortison wenig beachtet, es ist systemisch wirksamer als Hydrocortison aber weniger wirksam als das 6α-Methylprednisolon (J. Amer. Chem. Soc., 78, 1956, 6214).

Es wurde nun gefunden, dass die im Anspruch 1 gekennzeichneten Derivate des 6α-Methylhydrocortisons überraschenderweise bei topischer Applikation eine signifikant stärkere Wirksamkeit besitzen als die aus dem US-A- 3 141 876 und der Publikation J. Am. Chem. Soc. 78, 1956, 6213 vorbekannten Derivate des 6α-Methylhydrocortisons.

Die aus der US-A- 3 176 032 vorbekannten 6α-Methylsteroide tragen in der 11β-Position keinen Substituenten und besitzen demzufolge keine nennenswerte entzündungshemmende Wirksamkeit.

Diese Wirksamkeit ist oft sogar noch signifikant stärker als diejenige difluorierter Kortikoide, wie etwa das 6α, 9α-Difluor -11β- hydroxy -16α-methyl -21- valeryloxy -1,4- pregnadien -3,20-dion (= Nerisona).

Bei systemischer Applikation sind diese Derivate des 6α-Methylhydrocortisons überraschenderweise nicht signifikant stärker wirksam als die entsprechenden Derivate des Hydrocortisons.

Die neuen 6α-Methylhydrocortison-Derivate der allgemeinen Formel I gemäss Patentanspruch 1 eignen sich demzufolge in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001% bis 1% verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen 6α-Methylhydrocortison-Derivate können nach dem in den Patentansprüchen 26 und 27 gekennzeichneten Verfahren hergestellt werden, welche unter den Bedingungen durchgeführt werden können, wie sie in den deutschen Patentanmeldung DE-A- 16 18 599, 26 45 104, 26 45 105 und 23 40 591 und 19 58 954 sowie im US-A- 3 383 394 beschrieben sind.

Die nachfolgenden Ausführungsbeispiele dienen zur Erläuterung des erfindungsgemässen Verfahrens.

Beispiel 1

a) 100 g 17,21-Dihydroxy -6α- methyl -4- pregnen -3,20- dion und 10 g Pyridiniumtosylat werden in 700 ml Dimethylformamid bei Raumtemperatur unter Rühren gelöst und die klare Lösung mit 3,5 l Toluol verdünnt. Danach erwärmt man die Lösung in einem Glycerinbad und destilliert zur Entfernung von Wasserspuren bei einer Temperatur von 120 °C 1,2 l Toluol ab. In die heisse Reaktionslösung lässt man 240 ml Orthoessigsäuretrimethylester unter Rühren langsam zulaufen, lässt 30 Minuten reagieren und destilliert anschliessend 1 Stunde lang weiteres Toluol und andere leichtflüchtige Reaktionskomponenten ab. Man fügt 120 ml Pyridin hinzu, lässt auf 60 °C abkühlen und engt bei 70 °C Badtemperatur im Vakuum ein. Man verdünnt mit 400 ml Dimethylformamid und giesst die Lösung unter Rühren in 10 l Wasser ein. Hierbei fällt das Produkt in Form eines gelblich-weissen, kristallinen Niederschlages ab. Man rührt 2 Stunden nach, saugt ab, wäscht mit Wasser und trocknet 24 Stunden bei 40 °C im Vakuumtrockenschrank über Phosphorpentoxid. Man erhält 111,6 g 17,21-(1-Methoxy-ethylidendioxy) -6α- methyl -4- pregnen -3,20- dion vom Schmelzpunkt 85–87 °C.

b) Ein 7–14 Tage altes Bierwürze-Schrägröhrchen mit Curvularia lunata (NRRL 2380) wird mit 3 ml physiologischer Natriumchloridlösung abgeschwemmt und damit ein 2 l Erlenmeyerkolben beimpft, der 500 ml einer 30 Minuten bei 120 °C im Autoklaven sterilisierten Nährlösung aus 2% Glukose und 2% Corn steep, eingestellt auf pH 6,5, enthält. Nach 60 Stunden Schütteln auf einem Rotationsschüttler bei 30 °C dienen 250 ml dieser Anzuchtskultur zur Beimpfung des Vorfermenters. Ein 20 l-Vorfermenter, der mit 15 l eines bis 121 °C und 1 bar Überdruck sterilisierten Nährmediums der gleichen Zusammensetzung wie das Anzuchtmedium beschickt ist, wird mit 250 ml Anzuchtkultur beimpft. Unter Zugabe von Silicon SH als Antischaummittel wird nun bis 29 °C und 0,6 bar Druck unter Belüftung (15 l/Min.) und Rühren (220 U/Min) 24 Stunden germiniert. 1,5 Liter dieser Vorfermenter-Kultur dienen zur Beimpfung des Hauptfermenters. Ein 20 l-Hauptfermenter, der mit 13,5 l eines sterilisierten Nährmediums aus 3% Corn steep liquor und 0,7% Glukose, eingestellt auf pH 5,5, gefüllt ist, wird mit 1,5 l Vorfermenter-Kultur beimpft. Nach einer Anwachsphase von 12 Stunden unter Vorfermenterbedingungen wird sterilfiltrierte Lösung von 12,18 g 17,21-(1-Methoxy-ethylidendioxy) -6α-methyl -4- pregnen -3,20- dion in 130 ml Dimethylformamid hinzugegeben und weiter gerührt und belüftet. 4 Stunden nach der Substratzugabe wird der pH-Wert der Kulturbrühe auf pH 4,5 eingestellt und durch automatische Steuerung mittels 16%iger Natronlauge und 20%iger Schwefelsäure bis zum Ende der Fermentation auf diesem Wert ± pH 0,2 gehalten. Nach 51 Stunden Kontaktzeit ist die mikrobiologische Umwandlung vollständig. Der Fermenterinhalt wird nun in einer Durchlaufzentrifuge und das Kulturfiltrat als auch das abgeschleuderte Pilzmycel getrennt mit Methylisobutylketon extrahiert. Die Extrakte werden vereinigt und zunächst im Umlaufverdampfer bei 40 °C im Vakuum auf 1 Liter konzentriert, anschliessend im 2 l-Rundkolben am Rotationsverdampfer im Vakuum bei 40 °C Badtemperatur vollständig zur Trockne eingeengt. Der verbleibende ölige Rückstand wird mit 400 ml Hexan versetzt und nach kräftigem Schütteln dekantiert. Anschliessend versetzt man den Rückstand nochmals mit 400 ml Hexan und rührt 2 Stunden bei Raumtemperatur. Der nunmehr vollständig kristallisierte Rückstand wird abgesaugt, mit 100 ml Hexan gewaschen und 4 Stunden bei 60 °C im Vakuumtrockenschrank getrocknet. Man erhält 9,9 g 17-Acetoxy -11β,21- dihydroxy -6α- methyl -4- pregnen -3,20- dion, das nach Umkristallisation aus Aceton-Diisopropylether bei 192–194 °C schmilzt.

Beispiel 2

a) 15,0 g 11β,17,21-Trihydroxy -6α- methyl -4- pregnen -3,20- dion und 1,5 g Pyridiniumtosylat werden in 120 ml Dimethylformamid und 1050 ml Benzol (Toluol) unter Erwärmen gelöst. Bei einer Badtemperatur von 140 °C werden 450 ml Benzol (Toluol) über einen Wasserabscheider abdestilliert, die Lösung kurz auf 80 °C abgekühlt und mit 36 ml Orthopropionsäureethylester versetzt. Innerhalb von 20 Minuten destilliert man das restliche Benzol (Toluol) mit den leichtflüchtigen Lösungsmittelkomponenten ab. Nach Zugabe von 18 ml Pyridin wird im Vakuum eingeengt und 17,21- (1-Ethoxy-propylidendioxy) -11β- hydroxy -6α- methyl -4- pregnen -3,20- dion als Öl erhalten.

b) Eine Suspension obigen Rohproduktes in 450 ml Methanol wird mit einem Gemisch aus 18 ml 0,1 M wässriger Natriumacetat-Lösung und 162 ml 0,1 N wässriger Essigsäure 1 Stunde bei 100 °C refluxiert. Man engt bis zur Trübung ein, gibt auf Wasser und extrahiert mit viel Essigester. Die organische Phase wird mit Wasser gewaschen, über Natriumsulfat getrocknet und im Vakuum zur Trockne eingedampft. Das Rohprodukt wird an 650 g Kieselgel mit einem Methylenchlorid-Aceton-Gradienten (0–20% Aceton) gereinigt. Ausbeute 11,2 g 11β,21-Dihydroxy -6α- methyl -17- propionyloxy -4- pregnen -3,20- dion. Schmelzpunkt 197 °C.

Beispiel 3

a) Analog Beispiel 2a werden 15,0 g 11β, 17,21-Trihydroxy -6α- methyl -4- pregnen -3,20- dion mit Orthobuttersäuretrimethylester zu 11β-Hydroxy -17,21- (1-methoxy-butylidendioxy) -6α- methyl -4- pregnen -3,20- dion umgesetzt.

b) Das rohe 11β-Hydroxy -17,21- (1-methoxy-butylidendioxy) -6α- methyl -4- pregnen -3,20- dion wird analog Beispiel 2b mit einer Pufferlösung behandelt, aufgearbeitet und gereinigt. Ausbeute 11,4 g 17-Butyryloxy -11β,21- dihydroxy -6α- methyl -4- pregnen -3,20- dion. Schmelzpunkt 176 °C.

Beispiel 4

Zu einer Suspension von 9,3 g Kupfer(I)-jodid in 186 ml wasserfreiem Tetrahydrofuran werden bei 0 °C unter Argon 40 ml einer 5%igen Lösung von Methyllithium in Ether hinzugetropft. Die gelbe Lösung wird auf −30 °C abgekühlt und eine Lösung von 7,4 g 11β,17-Dihydroxy -6α- methyl -21- valeryloxy -4- pregnen -3,20- dion in 186 ml wasserfreiem Tetrahydrofuran zugesetzt. Man rührt 10 Minuten bei −25 °C weiter und gibt auf eine wässrige Ammoniumchloridlösung. Nach der Extraktion mit Methylenchlorid wird die organische Lösung gewaschen, über Natriumsulfat getrocknet und im Vakuum eingedampft. Das Rohprodukt wird an 600 g Kieselgel mit einem Methylenchlorid-Aceton-Gradienten (0–20% Aceton) gereinigt. Man isoliert 4,6 g 11β,21-Dihydroxy -6α- methyl -17- valeryloxy -4- pregnen -3,20- dion. Schmelzpunkt 160 °C.

Beispiel 5

7,7 g 11β,17-Dihydroxy -21- isobutyryloxy -6α- methyl -4- pregnen -3,20- dion werden analog Beispiel 4 mit Lithiumdimethylcuprat umlagert, aufgearbeitet und gereinigt. Man erhält 5,8 g 11β,21-Dihydroxy -17- isobutyryloxy -6α-methyl -4- pregnen -3,20- dion. Schmelzpunkt 191 °C.

Beispiel 6

Unter den Bedingungen des Beispiel 4 werden 7,3 g 11 β, 17-Dihydroxy -6α- methyl -21- trimethylacetoxy -4- pregnen -3,20- dion zu 1,7 g 11β,21-Dihydroxy -6α- methyl -17- trimethylacetoxy-4- pregnen -3,20- dion umlagert, aufgearbeitet und gereinigt.

Beispiel 7

a) 7,0 g 11β,17,21-Trihydroxy -6α- methyl -4- pregnen -3,20- dion werden analog Beispiel 2a mit Orthobenzoesäuretriethylester zu 17,21-(1-Ethoxy-benzylidendioxy) -11β- hydroxy -6α-methyl -4- pregnen -3,20- dion umgesetzt.

b) Das rohe 17,21-(1-Ethoxy-benzylidendioxy) -11β- hydroxy -6α- methyl -4- pregnen -3,20- dion wird unter den Bedingungen des Beispiels 2b hydrolysiert, aufgearbeitet und gereinigt. Ausbeute 4,6 g 17-Benzoyloxy -11β,21- dihydroxy -6α- methyl -4- pregnen -3,20- dion. Schmelzpunkt 214 °C.

Beispiel 8

Eine Lösung von 1,0 g 17-Acetoxy -11β,21-dihydroxy -6α- methyl -4- pregnen -3,20- dion in 20 ml Pyridin wird mit 3 ml Buttersäureanhydrid versetzt und 2 Stunden bei Raumtemperatur gerührt. Danach wird die Reaktionslösung in 150 ml gekühlte 8%ige Schwefelsäure einlaufen gelassen und weitere 5 Stunden gerührt, wobei das zunächst ölig ausgefallene Produkt durchkristallisiert. Man saugt ab, wäscht mit Wasser und trocknet 6 Stunden bei 80 °C im Vakuumtrockenschrank. Zur Reinigung wird das Rohprodukt aus Aceton-Diisopropylether umkristallisiert. Man erhält 940 mg 17-Acetoxy -21- butyryloxy -11β-hydroxy -6α- methyl -4- pregnen -3,20- dion vom Schmelzpunkt 98–102 °C.

Beispiel 9

Eine Lösung von 1,0 g 11β,21-Dihydroxy -6α-methyl -17- propionyloxy -4- pregnen -3,20-dion in 10 ml Pyridin wird mit 5 ml Propionsäureanhydrid 2 Stunden bei Raumtemperatur gerührt. Anschliessend gibt man auf eine Eiswasser-Natriumchlorid-Lösung, filtriert den Niederschlag ab und reinigt nach der üblichen Aufarbeitung das Rohprodukt an 65 g Kieselgel mit einem Methylenchlorid-Aceton-Gradienten (0–15% Aceton). Ausbeute 1,08 g 11β-Hydroxy -6α- methyl -17,21- dipropionyloxy -4- pregnen -3,20- dion. Schmelzpunkt 210 °C.

Beispiel 10

Analog Beispiel 8 wird 1,0 g 11β,21-Dihydroxy -6α- methyl -17- propionyloxy -4- pregnen -3,20- dion mit 5 ml Buttersäureanhydrid umgesetzt. Nach der Eiswasser-Natriumchlorid-Fällung extrahiert man mit Methylenchlorid, führt eine Wasserdampfdestillation aus und arbeitet wie üblich auf. Das Rohprodukt wird durch Kristallisation aus Aceton-Hexan gereinigt. Ausbeute 912 mg 21-Butyryloxy -11β- hydroxy -6α-methyl -17- propionyloxy -4- pregnen -3,20-dion. Schmelzpunkt 211 °C.

Beispiel 11

a) Eine Lösung von 5,0 g 21-Acetoxy -11β,17-dihydroxy -6α- methyl -4- pregnen -3,20- dion in 25 ml Pyridin wird bei −15 °C tropfenweise mit 3 ml Trifluoressigsäureanhydrid versetzt und 10 Minuten bei −10 °C gerührt. Man gibt auf eine Eiswasser-Natriumchloridlösung und filtriert den Niederschlag ab. Den Rückstand nimmt man in Methylenchlorid auf, wäscht neutral und engt nach dem Trocknen über Natriumsulfat im Vakuum ein. Ausbeute 5,1 g 21-Acetoxy -17- hydroxy -6α- methyl -11β- trifluoracetoxy -4- pregnen -3,20- dion.

b) 3,7 g des unter a) erhaltenen Rohproduktes werden in 45 ml Diethylenglykoldimethylether und 5,5 ml Propionsäureanhydrid mit 5,9 g 4-Dimethylaminopyridin 18 Stunden bei Raumtemperatur gerührt. Nach der üblichen Aufarbeitung isoliert man 3,9 g 21-Acetoxy -6α- methyl -17-propionyloxy -11β- trifluoracetoxy -4- pregnen -3,20- dion.

c) 2,0 g 21-Acetoxy -6α- methyl -17- propionyloxy -11β- trifluoracetoxy -4- pregnen -3,20- dion werden in 50 ml Methanol und 2,5 ml Triethylamin 4 Stunden bei Raumtemperatur gerührt. Man reinigt das Rohprodukt an 300 g Kieselgel mit einem Methylenchlorid-Aceton-Gradienten (0–15% Aceton) und isoliert 1,2 g 21-Acetoxy -11β- hydroxy -6α- methyl -17-propionyloxy -4- pregnen -3,20- dion.

Beispiel 12

Unter den Bedingungen des Beispiels 9 wird 1,0 g 17-Butyryloxy -11β,21- dihydroxy -6α-methyl -4- pregnen -3,20- dion mit Essigsäureanhydrid umgesetzt, aufgearbeitet und gereinigt. Ausbeute 1,02 g 21-Acetoxy -17- butyryloxy -11β- hydroxy -6α- methyl -4- pregnen -3,20-dion.

Beispiel 13

1,0 g 17-Butyryloxy -11β,21- dihydroxy -6α-methyl -4- pregnen -3,20- dion werden analog Beispiel 9 mit Propionsäureanhydrid umgesetzt, aufgearbeitet und gereinigt. Man erhält 1,1 g 17-Butyryloxy -11β- hydroxy -6α- methyl -21-propionyloxy -4- pregnen -3,20- dion. Schmelzpunkt 175 °C.

Beispiel 14

Man setzt 1,0 g 17-Butyryloxy -11β,21- dihydroxy -6α- methyl -4- pregnen -3,20- dion unter den Bedingungen des Beispiels 10 mit Buttersäureanhydrid um und arbeitet analog auf. Das Rohprodukt wird aus Aceton-Hexan umkristallisiert. Man isoliert 860 mg 17,21-Dibutyryloxy -11β- hydroxy -6α- methyl -4- pregnen -3,20-dion vom Schmelzpunkt 103 °C.

Beispiel 15

1,0 g 17-Butyryloxy -11β,21- dihydroxy -6α-methyl -4- pregnen -3,20- dion werden in Pyridin mit 5 ml Valeriansäureanhydrid, wie im Beispiel 10 beschrieben, umgesetzt. Nach üblicher Aufarbeitung und Reinigung an 65 g Kieselgel mit einem Methylenchlorid-Aceton-Gradienten (0–15% Aceton) isoliert man 980 mg 17-Butyryloxy -11β- hydroxy -6α- methyl -21- valeryloxy -4- pregnen -3,20- dion.

Beispiel 16

Unter den Bedingungen des Beispiels 10 wird 1,0 g 11β,21Dihydroxy -17- propionyloxy -6α-methyl -4- pregnen -3,20- dion mit Isobuttersäureanhydrid umgesetzt, aufgearbeitet und gereinigt. Ausbeute 870 mg 11β-Hydroxy -21- isobutyryloxy -6α- methyl -17- propionyloxy -4- pregnen -3,20- dion.

Beispiel 17

Analog Beispiel 9 wird 1,0 g 11β,21-Dihydroxy -6α- methyl -17- valeryloxy -4- pregnen -3,20-dion mit Acetanhydrid umgesetzt, aufgearbeitet und gereinigt. Man isoliert 980 mg 21-Acetoxy -11β- hydroxy -6α- methyl -17- valeryloxy -4-pregnen -3,20- dion.

Beispiel 18

2,9 g 11β,17-Dihydroxy -21- isovaleryloxy -6α- methyl -4- pregnen -3,20- dion werden analog Beispiel 4 mit Lithiumdimethylcuprat umlagert, aufgearbeitet und gereinigt. Man isoliert 1,6 g 11β,21-Dihydroxy -17- isovaleryloxy -6α-methyl -4- pregnen -3,20- dion.

Beispiel 19

Unter den Bedingungen des Beispiels 9 wird 1,0 g 11β,21-Dihydroxy -17- isobutyryloxy -6α-methyl -4- pregnen -3,20- dion mit Propionsäureanhydrid umgesetzt, aufgearbeitet und gereinigt. Man erhält 985 mg 11β-Hydroxy -17- isobutyryloxy -6α- methyl -21- propionyloxy -4-pregnen -3,20- dion.

Beispiel 20

Analog Beispiel 9 werden 2,2 g 17-Benzoyloxy -11β,21-dihydroxy -6α- methyl -4- pregnen -3,20- dion mit Essigsäureanhydrid umgesetzt und wie üblich aufgearbeitet. Das Rohprodukt wird an 350 g Kieselgel mit einem Methylenchlorid-Aceton-Gradienten (0–8% Aceton) gereinigt. Man isoliert 1,6 g 21-Acetoxy -17- benzoyloxy -11β- hydroxy -6α- methyl -4- pregnen -3,20-dion. Schmelzpunkt 210 °C.

Beispiel 21

Man setzt 1,0 g 11β,21-Dihydroxy -6α- methyl -17- trimethylacetoxy -4- pregnen -3,20- dion unter den Bedingungen des Beispiels 9 mit Propionsäureanhydrid um, arbeitet und reinigt, wie oben beschrieben auf. Man erhält 940 mg 11β-Hydroxy -6α- methyl -21- propionyloxy -17- trimethylacetoxy -4- pregnen -3,20- dion.

Beispiel 22

Unter den Bedingungen des Beispiels 8 werden 500 mg 17-Acetoxy -11β,21- dihydroxy -6α-methyl -4- pregnen -3,20- dion mittels 1,5 ml Essigsäureanhydrid zu 480 mg 17,21-Diacetoxy -11β- hydroxy -6α- methyl -4- pregnen -3,20-dion vom Schmelzpunkt 130–135 °C umgesetzt.

Beispiel 23

Unter den Bedingungen des Beispiels 8 werden 500 mg 17-Acetoxy -11β,21- dihydroxy -6α-methyl -4- pregnen -3,20- dion durch Reaktion mit 1,5 ml Propionsäureanhydrid in Pyridin zu 490 mg 17-Acetoxy -11β- hydroxy -6α- methyl -21- propionyloxy -4- pregnen -3,20- dion vom Schmelzpunkt 99–103 °C umgesetzt.

**Patentansprüche**

1. 6α-Methylhydrocortison-Derivate der allgemeinen Formel I

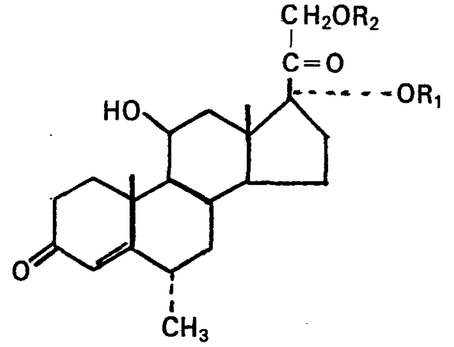

(I),

worin

$R_1$ ein 1-Oxoalkylrest mit 2 bis 6 Kohlenstoffatomen oder ein Benzoylrest und

$R_2$ ein Wasserstoffatom oder einen 1-Oxoalkylrest mit 2 bis 6 Kohlenstoffatomen darstellen.

2. 17α-Acetoxy -11β,21- dihydroxy -6α-methyl -4- pregnen -3,20- dion.

3. 11β,21-Dihydroxy -6α- methyl -17α- propionyloxy -4- pregnen -3,20- dion.

4. 17α-Butyryloxy -11β,21-dihydroxy -6α-methyl -4- pregnen -3,20- dion.

5. 11β,21-Dihydroxy -6α- methyl -17α- valeryloxy -4- pregnen -3,20- dion.

6. 11β,21-Dihydroxy -17α- isobutyryloxy -6α-methyl -4- pregnen -3,20- dion.

7. 11β,21-Dihydroxy -6α- methyl -17α-trimethylacetoxy -4- pregnen -3,20- dion.

8. 17α-Benzoyloxy -11β,21- dihydroxy -6α-methyl -4- pregnen -3,20- dion.

9. 17α-Acetoxy -21- butyryloxy -11β- hydroxy -6α- methyl -4- pregnen -3,20- dion.

10. 11β-Hydroxy -6α- methyl -17α,21- dipropionyloxy -4- pregnen -3,20- dion.

11. 21-Butyryloxy -11β- hydroxy -6α- methyl -17α- propionyloxy -4- pregnen -3,20- dion.

12. 21-Acetoxy -11β- hydroxy -6α- methyl -17α- propionyloxy -4- pregnen -3,20- dion.

13. 21-Acetoxy -17α- butyryloxy -11β- hydroxy -6α- methyl -4- pregnen -3,20- dion.

14. 17α- Butyryloxy -11β- hydroxy -6α- methyl -21- propionyloxy -4- pregnen -3,20- dion.

15. 17α,21-Dibutyryloxy -11β- hydroxy -6α-methyl -4- pregnen -3,20- dion.

16. 17α- Butyryloxy -11β- hydroxy -6α- methyl -21- valeryloxy -4- pregnen -3,20- dion.

17. 11β-Hydroxy -21- isobutyryloxy -6α-methyl -17α- propionyloxy -4- pregnen -3,20-dion.

18. 21-Acetoxy -11β- hydroxy -6α- methyl -17α- valeryloxy -4- pregnen -3,20- dion.

19. 11β,21-Dihydroxy -17α- isovaleryloxy -6α-methyl -4- pregnen -3,20- dion.

20. 11β-Hydroxy -17α- isobutyryloxy -6α- methyl -21- propionyloxy -4- pregnen -3,20- dion.

21. 21-Acetoxy -17α- benzoyloxy -11β- hydroxy -6α- methyl -4- pregnen -3,20- dion.

22. 11β-Hydroxy -6α- methyl -21- propionyloxy -17α- trimethylacetoxy -4- pregnen -3,20- dion.

23. 17α,21-Diacetoxy -11β- hydroxy -6α- methyl -4- pregnen -3,20- dion.

24. 17α-Acetoxy -11β- hydroxy -6α- methyl -21- propionyloxy -4- pregnen -3,20- dion.

25. Pharmazeutische Präparate gekennzeichnet durch den Gehalt an ein oder zwei 6α-Methylhydrocortison-Derivaten gemäss Anspruch 1 bis 24.

26. Verfahren zur Herstellung von 6α-Methylhydrocortison-Derivaten der allgemeinen Formel Ia

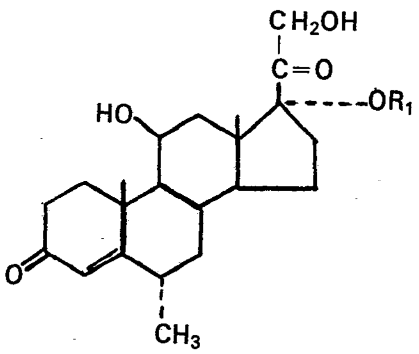

(I a),

worin

$R_1$ einen 1-Oxoalkylrest mit 2 bis 6 Kohlenstoffatomen bedeutet,

dadurch gekennzeichnet, dass man in an sich bekannter Weise

a) ein 6α-Methylsteroid-Derivat der allgemeinen Formel II oder III

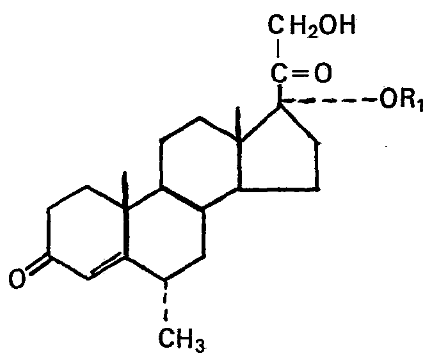

(II),

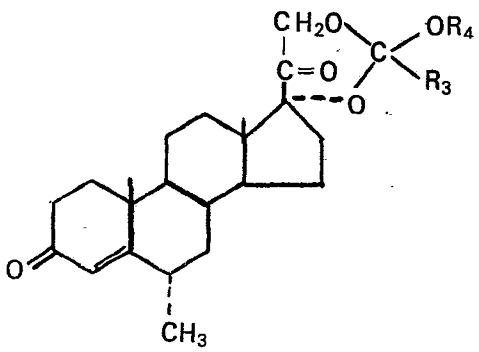

(III),

worin

$R_1$ die obengenannte Bedeutung besitzt

$R_3$ eine Alkylgruppe mit 1 bis 5 Kohlenstoffatomen oder eine Phenylgruppe und

$R_4$ eine Alkylgruppe mit 1 bis 4 Kohlenstoffatomen darstellen mit einer Pilzkultur der Gattung Curvularia bei einem pH-Wert von 4,0 bis 7,0 fermentiert, oder

b) einen 6α-Methylhydrocortison-orthocarbonsäureester der allgemeinen Formel IV

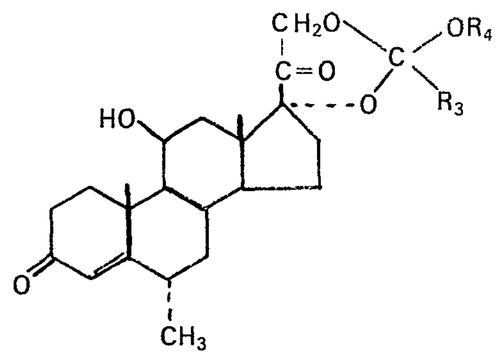

(IV),

worin

$R_3$ und $R_4$ die obengenannte Bedeutung besitzen hydrolysiert, oder

c) ein 6α-Methylhydrocortison -21- acylat der allgemeinen Formel V

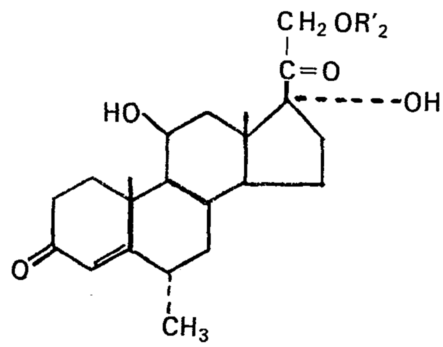

(V),

worin

$R'_2$ einen 1-Oxoalkylrest mit 2 bis 6 Kohlenstoffatomen bedeutet zu dem entsprechenden 17-Acylat umlagert.

27. Verfahren zur Herstellung von 6α-Methylhydrocortison-Derivaten der allgemeinen Formel Ib

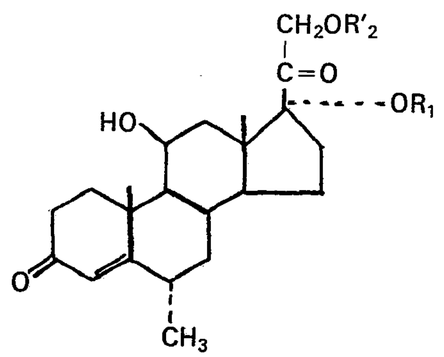

(Ib),

worin

$R_1$ einen Oxoalkylrest mit 2 bis 6 Kohlenstoffatomen oder einen Benzoylrest und

$R'_2$ einen 1-Oxoalkylrest mit 2 bis 6 Kohlenstoffatomen bedeuten, dadurch gekennzeichnet, dass man in an sich bekannter Weise

a) ein 6α-Methylhydrocortison -17- acylat der allgemeinen Formel Ia

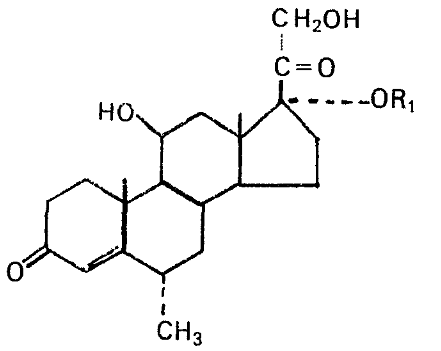

(I a),

worin

$R_1$ die obengenannte Bedeutung besitzt, in der 21-Position mit einer Alkancarbonsäure mit 2 bis 6 Kohlenstoffatomen, oder einem reaktionsfähigen Derivat derselben verestert, oder

b) ein 6α-Methylhydrocortison -21- acylat der allgemeinen Formel V

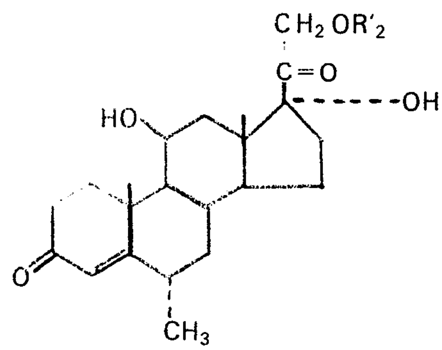

(V),

worin

$R'_2$ die obengenannte Bedeutung besitzt, in der 11-Position mit einer Trialkylsilylverbindung veräthert oder mit einem Derivat einer stark aciden Monocarbonsäure verestert, anschliessend die 17-Position mit einem Carbonsäurechlorid oder Carbonsäureanhydrid in Gegenwart von 4-Dimethylaminopyridin acyliert und die Schutzgruppe in der 11-Position abspaltet.

**Revendications**

1. Dérivés de la méthyl-6α hydrocortisone répondant à la formule générale I:

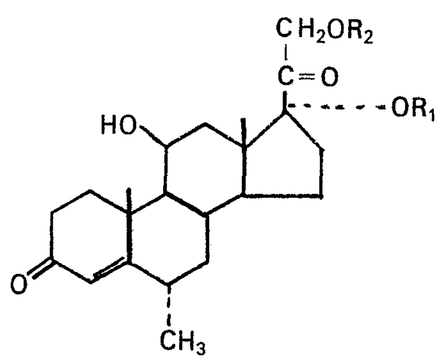

(I)

dans laquelle

$R_1$ représente un radical oxo-1 alkyle contenant de deux à six atomes de carbone ou un radical benzoyle et

$R_2$ représente un atome d'hydrogène ou un radical oxo-1 alkyle contenant de deux à six atomes de carbone.

2. L'acétoxy-17α dihydroxy-11β,21 méthyl-6α prégnène -4- dione-3,20.

3. La dihydroxy-11β,21 méthyl-6α propionyloxy-17α prégnène-4 dione-3,20.

4. La butyryloxy-17α dihydroxy-11β,21 méthyl-6α prégnène-4 dione-3,20.

5. La dihydroxy-11β,21 méthyl-6α valéryloxy-17α prégnène-4-dione-3,20.

6. La dihydroxy-11β, 21 isobutyryloxy-17α méthyl -6 prégnène -4 dione -3,20.

7. La dihydroxy -11β,21 méthyl -6α triméthylacétoxy -17α prégnène -4 dione -3,20.

8. La benzoyloxy -17α dihydroxy -11β,21 méthyl -6α prégnène -4 dione -3,20.

9. L'acétoxy -17α butyryloxy -21 hydroxy -11β méthyl -6α prégnène -4 dione -3,20.

10. L'hydroxy -11β méthyl -6α dipropionyloxy -17α,21 prégnène -4 dione -3,20.

11. La butyryloxy -21 hydroxy -11β méthyl -6α propionyloxy -17α prégnène -4 dione -3,20.

12. L'acétoxy-21 hydroxy-11β méthyl-6α propionyloxy -17α prégnène -4 dione -3,20.

13. L'acétoxy -21 butyryloxy -17α hydroxy -11β méthyl -6α prégnène -4 dione -3,20.

14. La butyryloxy -17α hydroxy -11β méthyl -6α propionyloxy -21 prégnène -4 dione -3,20.

15. La dibutyryloxy -17α,21 hydroxy -11β méthyl -6α prégnène -4 dione -3,20.

16. La butyryloxy -17α hydroxy -11β méthyl -6α valéryloxy -21 prégnène -4 dione -3,20.

17. L'hydroxy -11β isobutyryloxy -21 méthyl -6α propionyloxy -17α prégnène -4 dione -3,20.

18. L'acétoxy -21 hydroxy -11β méthyl -6α valéryloxy -17α prégnène -4 dione -3,20.

19. La dihydroxy -11β,21 isovaléryloxy -17α méthyl -6α prégnène -4 dione -3,20.

20. L'hydroxy -11β isobutyryloxy -17α méthyl -6α propionyloxy -21 prégnène -4 dione -3,20.

21. L'acétoxy -21 benzoyloxy -17α hydroxy -11β méthyl -6α prégnène -4 dione -3,20.

22. L'hydroxy -11β méthyl -6α propionyloxy -21 triméthylacétoxy -17α prégnène -4 dione -3,20.

23. La diacétoxy -17α,21 hydroxy -11β méthyl -6α prégnène -4 dione -3,20.

24. L'acétoxy -17α hydroxy -11β méthyl -6α propionyloxy -21 prégnène -4 dione -3,20.

25. Médicaments caractérisés en ce qu'ils contiennent un ou deux dérivés de la méthyl -6α hydrocortisone selon l'une quelconque des revendications 1 à 24.

26. Procédés de préparation de dérivés de la méthyl -6α hydrocortisone répondant à la formule générale Ia:

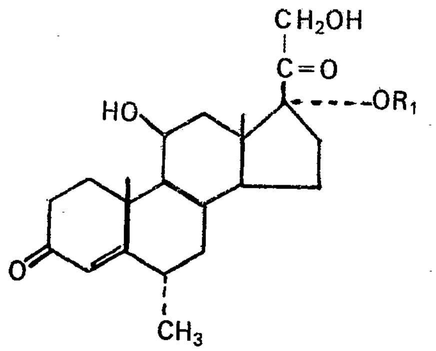

(I a),

dans laquelle

$R_1$ représente un radical oxo -1 alkyle contenant de deux à six atomes de carbone,

procédé caractérisé en ce que, en opérant de manière connue:

a) on fait fermenter un méthyl -6 stéroïde répondant à l'une des formules générales II et III:

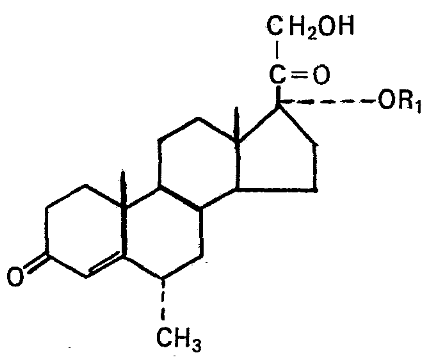

(II),

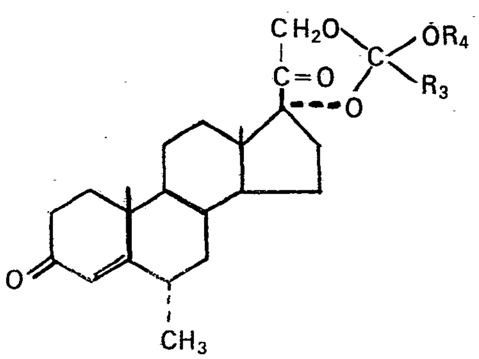

(III),

dans lesquelles

$R_1$ a la signification indiquée ci-dessus,

$R_3$ représente un radical alkyle contenant de 1 à 5 atomes de carbone ou un radical phényle et

représente un radical alkyle contenant de 1 à 4 atomes de carbone,

avec une culture d'un mycète de l'espèce Curvularia, à un pH de 4,0 à 7,0, ou

b) on hydrolyse un ester orthocarboxylique de la méthyl -6α hydrocortisone répondant à la formule générale IV:

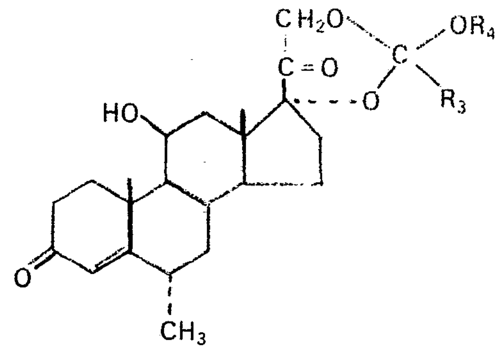

(IV)

dans laquelle

$R_3$ et $R_4$ ont les significations précédemment données, ou

c) on transpose un acylate en 21 de la méthyl -6α hydrocortisone répondant à la formule générale V:

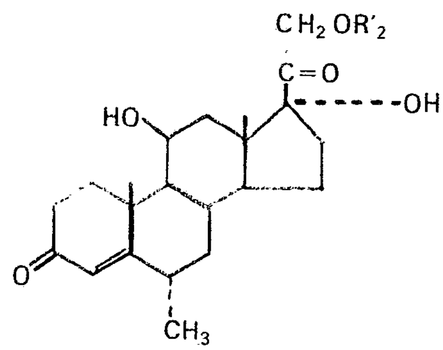

(V),

dans laquelle

$R'_2$ représente un radical oxo -1 alkyle contenant de 2 à 6 atomes de carbone,

de manière à obtenir l'acylate en 17 correspondant.

27. Procédé de préparation de dérivés de la méthyl -6α hydrocortisone répondant à la formule générale Ib:

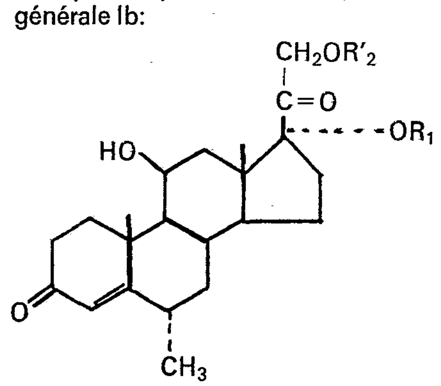

(Ib),

dans laquelle

$R_1$ représente un radical oxo-alkyle contenant de 2 à 6 atomes de carbone ou un radical benzoyle et

$R'_2$ représente un radical oxo -1 alkyle contenant de 2 à 6 atomes atomes de carbone,

procédé caractérisé en ce que, en opérant de manière connue:

a) on estérifie un acylate en 17 de la méthyl -6α hydrocortisone répondant à la formule générale Ia:

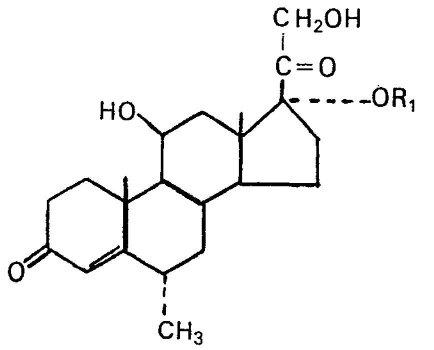

(I a),

dans laquelle

$R_1$ a la signification précédemment donnée, en position 21, avec un acide alcane-carboxylique contenant de 2 à 6 atomes de carbone, ou avec un dérivé réactif d'un tel acide, ou

b) on éthérifie un acylate en 21 de la méthyl -6α hydrocortisone répondant à la formule générale V:

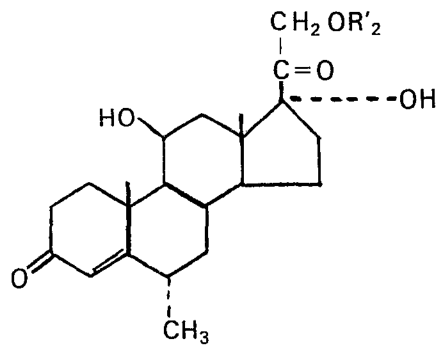

(V),

dans laquelle

$R'_2$ a la signification précédemment donnée, en position 11, avec un composé trialkylsilylique, ou on l'estérifie avec un dérivé d'un acide monocarboxylique très acide, puis on acyle en position 17 avec un chlorure d'acide carboxylique ou un anhydride d'acide carboxylique en présence de diméthylamino -4 pyridine et on élimine le radical protecteur de la position 11.

**Claims**

1. 6α-methylhydrocortisone derivatives of the general formula I

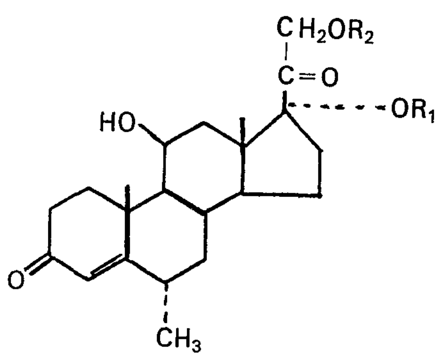

(I)

in which

$R_1$ represents a 1-oxoalkyl radical having from 2 to 6 carbon atoms or a benzoyl radical, and

$R_2$ represents a hydrogen atom or a 1-oxoalkyl radical having from 2 to 6 carbon atoms.

2. 17α-acetoxy -11β,21 dihydroxy -6α -methyl -4- pregnen -3,20- dione.

3. 11β,21-dihydroxy -6α- methyl -17α- propionyloxy -4- pregnen -3,20- dione.

4. 17α-butyryloxy -11β,21 - dihydroxy -6α-methyl -4- pregnen -3,20- dione.

5. 11β,21-dihydroxy -6α- methyl -17α- valeryloxy -4- pregnen -3,20- dione.

6. 11β,21-dihydroxy -17α- isobutyryloxy -6α-methyl -4- pregnen -3,20- dione.

7. 11β,21-dihydroxy -6α- methyl -17α- trimethylacetoxy -4- pregnen -3,20- dione.

8. 17α-benzoyloxy -11β,21 - dihydroxy -6α-methyl -4- pregnen -3,20- dione.

9. 17α-acetoxy -21 - butyryloxy -11β- hydroxy -6α- methyl -4- pregnen -3,20- dione.

10. 11β-hydroxy -6α- methyl -17α,21 - dipropionyloxy -4- pregnen -3,20- dione.

11. 21-butyryloxy -11β- hydroxy -6α- methyl -17α- propionyloxy -4- pregnen -3,20- dione.

12. 21-acetoxy -11β- hydroxy -6α- methyl -17α- propionyloxy -4- pregnen -3,20- dione.

13. 21-acetoxy -17α- butyryloxy -11β- hydroxy -6α- methyl -4- pregnen -3,20- dione.

14. 17α-butyryloxy -11β- hydroxy -6α- methyl -21 - propionyloxy -4- pregnen -3,20- dione.

15. 17α,21-dibutyryloxy -11β- hydroxy -6α-methyl -4- pregnen -3,20- dione.

16. 17α-butyryloxy -11β- hydroxy -6α- methyl -21 - valeryloxy -4- pregnen -3,20- dione.

17. 11β-hydroxy -21 - isobutyryloxy -6α-methyl -17α- propionyloxy -4- pregnen -3,20-dione.

18. 21-acetoxy -11β- hydroxy -6α- methyl -17α- valeryloxy -4- pregnen -3,20- dione.

19. 11β,21-dihydroxy -17α- isovaleryloxy -6α-methyl -4- pregnen -3,20- dione.

20. 11β-hydroxy -17α- isobutyryloxy -6α-methyl -21 - propionyloxy -4- pregnen -3,20-dione.

21. 21-acetoxy -17α- benzoyloxy -11β- hydroxy -6α- methyl -4- pregnen -3,20- dione.

22. 11β-hydroxy -6α- methyl -21 - propionyloxy -17α- trimethylacetoxy -4- pregnen -3,20-dione.

23. 17α,21-diacetoxy -11β- hydroxy -6α-methyl -4- pregnen -3,20- dione.

24. 17α-acetoxy -11β- hydroxy -6α-methyl -21 - propionyloxy -4- pregnen -3,20- dione.

25. Pharmaceutical preparations characterised by a content of one or two 6α-methylhydrocortisone derivatives according to claims 1 to 24.

26. Process for the manufacture of 6α-methylhydrocortisone derivatives of the general formula Ia

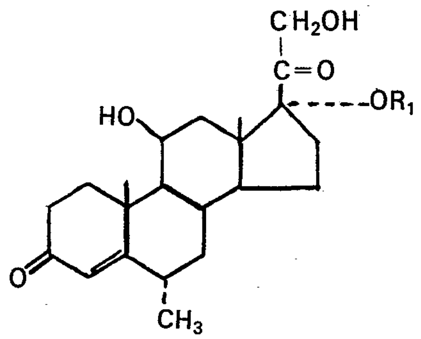

(I a),

in which

$R_1$ represents a 1-oxoalkyl radical having from 2 to 6 carbon atoms, characterised in that, in a manner known per se,

a) a 6α-methylsteroid derivative of the general formula II or III

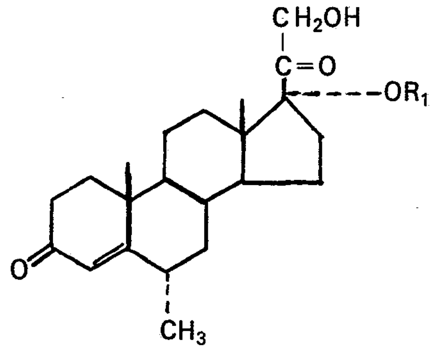

(II),

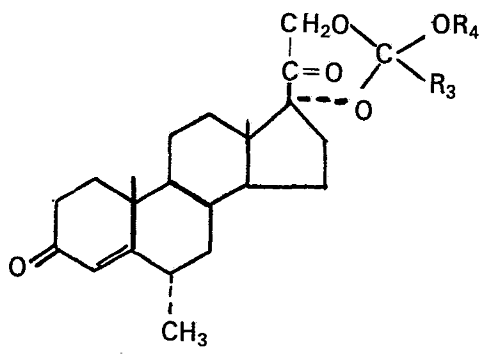

(III),

in which

$R_1$ has the meaning given above,

$R_3$ represents an alkyl group having from 1 to 5 carbon atoms or a phenyl group, and

$R_4$ represents an alkyl group having from 1 to 4 carbon atoms

is fermented with a fungal culture of the genus Curvularia at a pH value of from 4.0 to 7.0, or

b) a 6α-methylhydrocortisone orthocarboxylic acid ester of the general formula IV

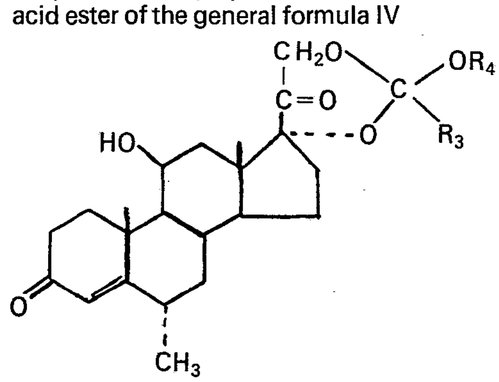

(IV),

in which

$R_3$ and $R_4$ have the meanings given above is hydrolysed, or

c) a 6α-methylhydrocortisone 21-acylate of the general formula V

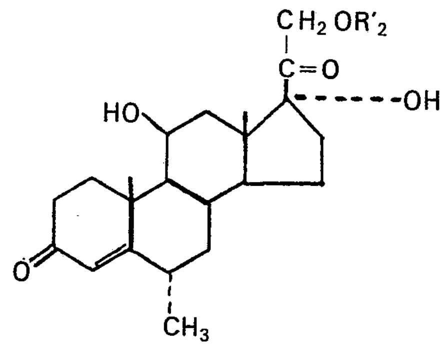

(V),

in which

$R'_2$ represents a 1-oxoalkyl radical having from 2 to 6 carbon atoms is transposed to form the corresponding 17-acylate.

27. Process for the manufacture of 6α-methylhydrocortisone derivatives of the general formula Ib

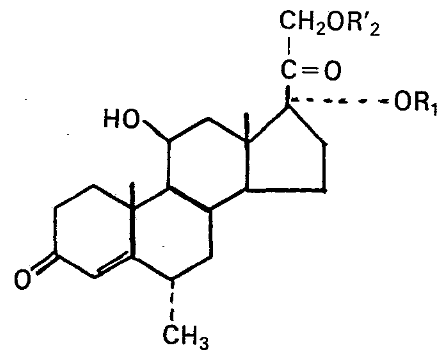

(Ib),

in which

$R_1$ represents an oxoalkyl radical having from 2 to 6 carbon atoms or a benzoyl radical, and

$R'_2$ represents a 1-oxoalkyl radical having from 2 to 6 carbon atoms, characterised in that, in a manner known per se,

a) a 6α-methylhydrocortisone 17-acylate of the general formula Ia

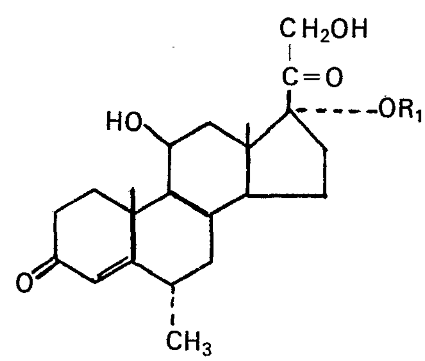

(I a),

in which

$R_1$ has the meaning given above is esterified in the 21-position by an alkanecarboxylic acid having from 2 to 6 carbon atoms or a reactive derivative thereof, or

b) a 6α-methylhydrocortisone 21-acylate of the general formula V

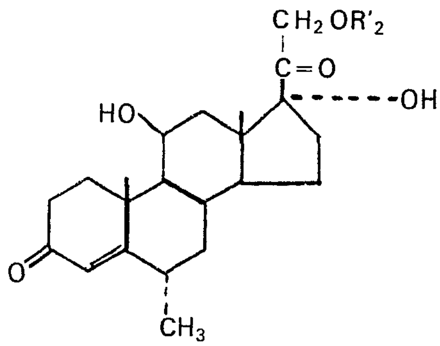

(V),

in which

$R'_2$ has the meaning given above is etherified in the 11-position by a trialkylsilyl compound or is esterified in the 11-position by a derivative of a strongly acidic monocarboxylic acid, subsequently the 17-position is acylated by a carboxylic acid chloride or carboxylic acid anhydride in the presence of 4-dimethylaminopyridine and-the protecting group in the 11-position is split off.